(19) Europäisches Patentamt
European Patent Office
Office européen des brevets

(11) **EP 2 780 699 B1**

(12) **FASCICULE DE BREVET EUROPEEN**

(45) Date de publication et mention de la délivrance du brevet:
**14.10.2015 Bulletin 2015/42**

(21) Numéro de dépôt: **12794408.0**

(22) Date de dépôt: **30.10.2012**

(51) Int Cl.:
*G01N 29/26* (2006.01)   *G01N 29/44* (2006.01)
*G01N 29/06* (2006.01)   *G01B 17/06* (2006.01)

(86) Numéro de dépôt international:
**PCT/FR2012/052507**

(87) Numéro de publication internationale:
**WO 2013/072593 (23.05.2013 Gazette 2013/21)**

(54) **PROCÉDÉ DE RECONSTRUCTION DE LA GÉOMÉTRIE D'UNE SURFACE D'UN OBJET PAR SONDAGE ÉCHOGRAPHIQUE, PROGRAMME D'ORDINATEUR CORRESPONDANT ET DISPOSITIF DE SONDAGE À ULTRASONS**

VERFAHREN ZUR REKONSTRUKTION DER GEOMETRIE EINER OBERFLÄCHE EINES OBJEKTS DURCH ECHOLOTEN, ENTSPRECHENDES COMPUTERPROGRAMM UND VORRICHTUNG ZUR ULTRASCHALLPRÜFUNG

METHOD FOR RECONSTRUCTING THE GEOMETRY OF A SURFACE OF AN OBJECT VIA ECHOGRAPHIC SOUNDING, CORRESPONDING COMPUTER PROGRAM AND DEVICE FOR ULTRASONIC SOUNDING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **15.11.2011  FR 1160399**

(43) Date de publication de la demande:
**24.09.2014  Bulletin 2014/39**

(73) Titulaire: **Commissariat à l'Énergie Atomique et aux Énergies Alternatives**
**75015 Paris (FR)**

(72) Inventeurs:
• **ROBERT, Sébastien**
 **F-93400 Saint-Ouen (FR)**
• **CASULA, Olivier**
 **F-91310 Longpont-sur-Orge (FR)**
• **IAKOVLEVA, Ekaterina**
 **91470, Limours (FR)**

(74) Mandataire: **Bonnet, Michel**
**Cabinet Bonnet**
**93, rue Réaumur - Boîte 10**
**75002 Paris (FR)**

(56) Documents cités:
**EP-A1- 0 486 689     US-A- 5 750 895**

• **S ROBERT ET AL: "MÉTHODES ULTRASONORES DE DÉTECTION ET D'IMAGERIE TEMPS-RÉEL IMPLÉMENTÉES DANS LES SYSTÈMES D'ACQUISITION M2M - REAL-TIME ULTRASONIC TECHNIQUES IMPLEMENTED IN M2M ACQUISITION SYSTEMS", JOURNÉES COFREND "CONFÉRENCES ET EXPOSITION SUR LES ESSAIS NON DESTRUCTIFS" 2011, 24 mai 2011 (2011-05-24), pages 1-13, XP055037304, Dunkerque (FR) cité dans la demande**

**Description**

La présente invention concerne un procédé de reconstruction de la géométrie d'une surface d'un objet par sondage échographique à l'aide d'une sonde à ultrasons munie d'une pluralité de transducteurs. Elle concerne également un programme d'ordinateur et une sonde à ultrasons pour la mise en oeuvre de ce procédé.

L'invention s'applique notamment au domaine du contrôle non destructif de pièces mécaniques ayant une forme complexe, en particulier lors d'un contrôle en immersion, dans lequel une pièce mécanique est plongée dans un liquide pour être sondée à distance, par exemple dans le secteur aéronautique. Mais elle peut également s'appliquer lors d'un contrôle par contact direct entre la sonde et la pièce mécanique à sonder lorsqu'il est souhaité de déterminer une surface complexe du fond de cette pièce. Plus généralement, elle concerne des domaines d'application variés et peut être utilisée dès lors qu'il est souhaité de reconstruire la géométrie d'un objet ou d'une interface à l'aide d'ondes ultrasonores et d'une sonde à transducteurs multiples. On peut par exemple citer le domaine médical, celui de l'acoustique sous-marine, des sonars, etc.

Des méthodes de sondage par balayage électronique sont connues pour déterminer finement la surface *a priori* inconnue d'un objet. Malheureusement, ces méthodes étant basées sur une contrainte de traitements successifs et indépendants réalisés par les transducteurs de la sonde, chaque transducteur devant attendre, avant d'émettre un signal, que le transducteur précédent ait traité l'écho de son propre signal, le traitement global de la surface est long. Ces méthodes ne sont par conséquent pas adaptées à des systèmes embarqués pour des contrôles à grande vitesse. En outre, ces méthodes étant basées sur le traitement de signaux issus chaque fois d'un unique transducteur, les échos de surface renvoyés lors d'un sondage échographique peuvent être d'amplitude insuffisante pour effectuer des mesures fiables ou complètes.

Il est alors préféré un procédé dit « temps réel », c'est-à-dire ne présentant pas cette contrainte, même si les émissions peuvent être réalisées par les transducteurs en suivant une certaine loi de retards.

Ainsi, l'invention concerne plus particulièrement un procédé de reconstruction de la géométrie d'une surface d'objet comprenant les étapes suivantes :

- commander les transducteurs afin qu'ils émettent vers ladite surface de l'objet des ondes ultrasonores présentant des retards d'émission initiaux les unes par rapport aux autres,
- exécuter au moins une fois la boucle d'étapes suivante, de manière à obtenir après au moins une itération un front d'onde reçu simultanément sur la surface :

  • recevoir depuis les transducteurs des signaux de mesure intermédiaires, mesurant en particulier des échos dus à des réflexions des ondes ultrasonores sur ladite surface de l'objet,
  • corriger les retards d'émission des transducteurs à l'aide des signaux de mesure intermédiaires et commander les transducteurs afin qu'ils émettent vers ladite surface de l'objet des ondes ultrasonores présentant les retards d'émission corrigés les unes par rapport aux autres,

- recevoir depuis les transducteurs des signaux de mesure finaux résultant de la réflexion d'un front d'onde reçu simultanément sur ladite surface de l'objet.

Un procédé de ce type est par exemple décrit dans la demande de brevet publiée sous le numéro US 2006/0195273 A1.

Plus précisément, ce document décrit un procédé dans lequel la boucle n'est exécutée qu'une seule fois et selon lequel l'étape de correction des retards d'émission initiaux à partir des signaux de mesure intermédiaires consiste à procéder en deux temps : tout d'abord, une estimation de la surface inconnue de l'objet est calculée de façon explicite à partir des signaux de mesure intermédiaires obtenus à l'occasion d'un premier tir ; puis une loi de retards est calculée à partir de ce contour et appliquée à l'occasion d'un second tir.

Plus précisément également, dans ce document, les retards d'émission initiaux sont des retards nuls. Au second tir, il n'y a donc pas explicitement de correction des retards d'émission initiaux mais l'application directe de la loi de retards établie à partir de la surface estimée de l'objet.

Ce procédé « temps réel » a été mis au point pour le contrôle de structures complexes en composites stratifiés, c'est-à-dire une matrice de résine organique renforcée avec des tissus de fibres de carbone, et s'applique en particulier à la détection de défauts de type délaminage d'orientation quasi parallèle à la surface de la pièce. Il permet de former un front d'onde incident de même courbure que la surface de la pièce. L'onde est alors transmise dans la pièce en incidence normale en tous points de la surface, ce qui optimise la détection de défauts par rapport à une émission ultrasonore non adaptée à la géométrie de la pièce. La détection et la localisation des défauts s'effectuent par l'analyse du B-scan obtenu (représentation cumulée des N signaux de mesure finaux reçus par les N transducteurs de la sonde). Ce procédé est particulièrement adapté aux matériaux composites stratifiés pour lesquels les plis ont une orientation quasi parallèle à la surface et participent, de fait, à la dégradation de l'onde ultrasonore transmise si celle-ci n'est pas adaptée à la géométrie de l'objet. De plus, ce principe est bien adapté à cette problématique car les défauts recherchés sont des délaminages entre des plis du composite et ont donc des géométries quasi parallèles à la surface de l'objet. En outre, ce procédé n'est pas restreint à ce type de matériaux, il peut aussi être appliqué pour le contrôle d'autres matériaux, par exemple métalliques.

Enfin, ce procédé a été généralisé pour contrôler des objets présentant des géométries plus complexes et, en particulier, pour s'adapter à n'importe quel type de surface, qu'elle soit concave, convexe ou plane avec une forte inclinaison. En effet, lorsque la surface à déterminer de l'objet présente d'importantes variations de géométrie, de fortes interférences entre les ondes émises par les transducteurs puis réfléchies par l'objet subsistent et altèrent encore le B-scan, même après application du procédé décrit dans la demande de brevet US 2006/0195273 A1.

Ainsi, dans l'article de S. Robert et al, intitulé « Méthodes ultrasonores de détection et d'imagerie temps-réel implémentées dans les systèmes d'acquisition M2M », publié à l'occasion des journées Cofrend 2011, « conférences et exposition sur les essais non destructifs », du 24 au 27 mai 2011 à Dunkerque (FR), plus précisément au chapitre 3 de ce document, il est proposé de répéter la boucle d'étapes précitée de manière à converger par itérations successives vers une réception vraiment simultanée du front d'onde sur la surface à déterminer en s'affranchissant des interférences y compris en présence d'une surface très complexe. Le B-scan obtenu est alors de bien meilleure qualité et permet notamment une meilleure détection des défauts éventuels. En général, 4 à 5 itérations de la boucle d'étapes peuvent suffire pour parvenir à un résultat convenable quel que soit le type de géométrie inspectée. Ainsi, le procédé permet d'inspecter les différentes géométries (planes avec ou sans inclinaison, concaves, convexes) d'un même objet en utilisant une sonde unique, par exemple une sonde conventionnelle avec une géométrie plane de ses transducteurs.

En revanche, du fait de la loi de retards finale appliquée, ce B-scan donne une représentation déformée de l'objet sondé, en particulier de la surface atteinte simultanément par le front d'onde puisque celle-ci se présente alors logiquement sous une forme plane. La représentation brute des résultats d'acquisition sous la forme d'un B-scan ne suffit pas pour localiser et caractériser un défaut avec précision. Pour ce faire, le procédé doit inclure une connaissance de la surface de l'objet.

Il peut ainsi être souhaité de prévoir un procédé de détermination d'une surface d'un objet par sondage échographique qui permette de s'affranchir d'au moins une partie des problèmes et contraintes indiqués ci-dessus, mais qui reste « temps réel » au sens précité.

Il est donc proposé un procédé de reconstruction de la géométrie d'une surface d'un objet par sondage échographique à l'aide d'une sonde à ultrasons munie d'une pluralité de transducteurs, comprenant les étapes suivantes :

- commander les transducteurs afin qu'ils émettent vers ladite surface de l'objet des ondes ultrasonores présentant des retards d'émission initiaux les unes par rapport aux autres,
- exécuter au moins une fois la boucle d'étapes suivante, de manière à obtenir après au moins une itération un front d'onde reçu simultanément sur la surface :

  • recevoir depuis les transducteurs des signaux de mesure intermédiaires, mesurant en particulier des échos dus à des réflexions des ondes ultrasonores sur ladite surface de l'objet,
  • corriger les retards d'émission des transducteurs à l'aide des signaux de mesure intermédiaires et commander les transducteurs afin qu'ils émettent vers ladite surface de l'objet des ondes ultrasonores présentant les retards d'émission corrigés les unes par rapport aux autres,

- recevoir depuis les transducteurs des signaux de mesure finaux résultant de la réflexion d'un front d'onde reçu simultanément sur ladite surface de l'objet,

ce procédé comportant en outre les étapes suivantes :

- détermination de temps de vol spéculaires entre chaque transducteur et ladite surface de l'objet à partir des signaux de mesure finaux et des retards d'émission corrigés, et
- reconstitution géométrique de ladite surface de l'objet à partir des temps de vol spéculaires déterminés.

Par « temps de vol spéculaire » entre un transducteur et la surface de l'objet, on entend le temps minimal mis par un signal émis par ce transducteur pour revenir sous forme d'écho suite à une réflexion contre la surface de l'objet. Le temps de vol spéculaire est alors significatif de la plus courte distance séparant le transducteur de la surface de l'objet, c'est-à-dire du segment de droite normal à cette surface et la reliant au transducteur.

Ainsi, en exploitant astucieusement le résultat de la mesure finale réalisée par les transducteurs pour déterminer les temps de vol spéculaires liés à chaque transducteur, selon le type de sonde à transducteurs multiples utilisée, linéaire ou respectivement matricielle, il est possible de reconstituer finement la géométrie, bidimensionnelle ou respectivement tridimensionnelle, de la surface détectée.

De façon optionnelle, la détermination des temps de vol spéculaires comporte :

- la détermination d'un temps de vol aller retour, commun à tous les transducteurs, du front d'onde reçu simultanément sur ladite surface de l'objet, et
- le calcul des temps de vol spéculaires à partir de ce temps de vol aller retour, des retards d'émission corrigés et de décalages de réception appliqués aux transducteurs.

De façon optionnelle également, la reconstitution géométrique de ladite surface comporte :

- la détermination de distances séparant chaque transducteur de ladite surface à partir des temps de vol spéculaires déterminés,
- le calcul de coordonnées de points de ladite surface en fonction de coordonnées des transducteurs et des distances déterminées, et
- la reconstitution géométrique de ladite surface par interpolation entre ces points.

De façon optionnelle également, le calcul des coordonnées de points de ladite surface est basé sur l'hypothèse que ladite surface est tangentielle à un ensemble de sphères centrées respectivement sur les transducteurs et de rayons respectifs correspondant aux distances déterminées.

De façon optionnelle également, les transducteurs sont disposés linéairement et la reconstitution géométrique de ladite surface comporte la reconstitution d'un profil de cette surface dans le plan de contrôle de la sonde.

Par « plan de contrôle de la sonde », qui est une terminologie connue dans le domaine du contrôle non destructif, on entend le plan principal d'émission des transducteurs.

De façon optionnelle également, N transducteurs sont disposés linéairement, les coordonnées de leurs centres pouvant s'exprimer sous la forme $(c_n, 0)$ dans un repère $(O, x, y)$ lié à la sonde, et dans lequel les coordonnées $(x_n, y_n)$ desdits points de ladite surface sont calculées à l'aide de la relation suivante : $\forall n, 1 \leq n \leq N\text{-}1, \begin{cases} x_n = c_n - d_n \cdot d_n' \\ y_n = d_n \sqrt{1 - d_n'} \end{cases}$ , où

$d_n' = \dfrac{d_{n+1} - d_n}{c_{n+1} - c_n}$ et $d_n$ désigne la distance séparant le n-ième transducteur de ladite surface.

De façon optionnelle également, les transducteurs sont disposés bidimensionnellement en matrice et la reconstitution géométrique de ladite surface comporte une reconstitution tridimensionnelle de cette surface.

De façon optionnelle également, la reconstitution géométrique de ladite surface se fait par interpolation linéaire ou bilinéaire entre lesdits points.

L'invention a également pour objet un programme d'ordinateur téléchargeable depuis un réseau de communication et/ou enregistré sur un support lisible par ordinateur et/ou exécutable par un processeur, comprenant des instructions pour l'exécution des étapes d'un procédé de reconstruction de la géométrie d'une surface d'un objet par sondage échographique selon l'invention, lorsque ledit programme est exécuté sur un ordinateur.

Enfin, l'invention a également pour objet un dispositif de sondage à ultrasons comportant :

- une sonde comprenant un boîtier et une pluralité de transducteurs à ultrasons attachés au boîtier,
- des moyens de commande et de traitement conçus pour mettre en oeuvre un procédé de reconstruction de la géométrie d'une surface d'un objet selon l'invention.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif de sondage à ultrasons selon un mode de réalisation de l'invention,
- la figure 2 illustre les étapes successives d'un procédé de détermination de surface d'objet mis en oeuvre par le dispositif de sondage de la figure 1,
- la figure 3 représente un premier exemple d'utilisation de la sonde de la figure 1 pour sonder un objet dont la géométrie est représentative d'un bourrelet de soudure,
- les figures 4 à 7 sont des B-scan obtenus à partir de signaux de mesure sur l'objet à bourrelet de soudure de la figure 3, pour respectivement une première, une deuxième, une troisième et une quatrième itération d'une boucle d'étapes du procédé de la figure 2,

- les figures 8A, 8B et 8C illustrent, à l'aide de diagrammes et d'une vue en coupe de l'objet à bourrelet de soudure de la figure 3, le fonctionnement d'étapes de calcul de temps de vol et trajets spéculaires du procédé de la figure 2,
- la figure 9 illustre, à l'aide d'une vue en coupe de l'objet à bourrelet de soudure de la figure 3, le fonctionnement d'étapes de reconstitution géométrique de surface du procédé de la figure 2,
- la figure 10 illustre, à l'aide d'une vue en coupe de l'objet à bourrelet de soudure de la figure 3, une première application du procédé de la figure 2,
- la figure 11 illustre, à l'aide d'une vue en coupe de l'objet à bourrelet de soudure de la figure 3, une deuxième application du procédé de la figure 2,
- la figure 12 représente un second exemple d'utilisation de la sonde de la figure 1 pour sonder un objet de type rayon de composite,
- les figures 13 à 16 sont des B-scan obtenus à partir de signaux de mesure sur l'objet composite de la figure 12, pour respectivement une première, une deuxième, une troisième et une quatrième itération d'une boucle d'étapes du procédé de la figure 2, et
- la figure 17 illustre, à l'aide d'un B-scan et d'une vue en coupe de l'objet composite de la figure 12, une troisième application du procédé de la figure 2.

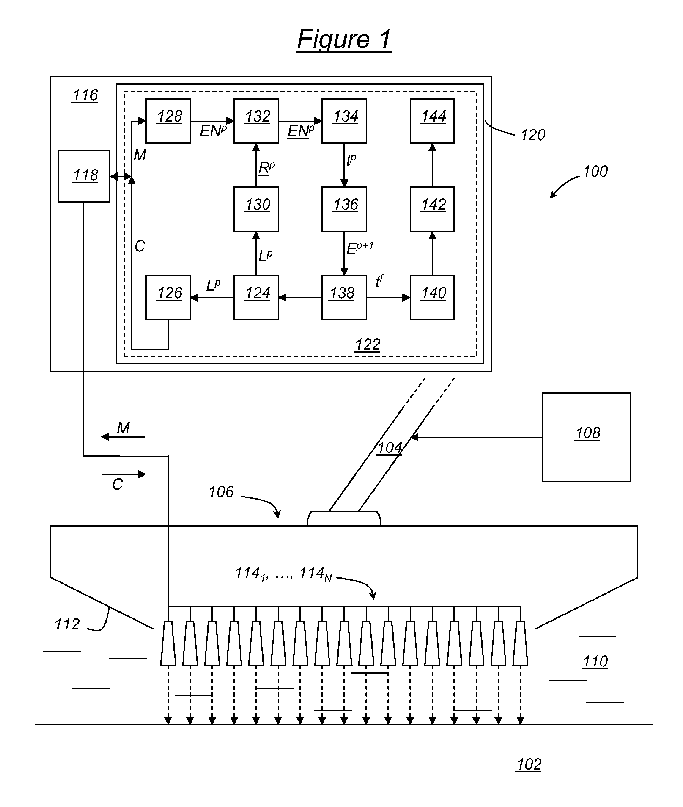

En référence à la figure 1, un dispositif de sondage 100 d'un objet 102 selon un mode de réalisation de l'invention comporte un bras articulé 104, une sonde à ultrasons 106 fixée au bras articulé 104 et des moyens 108 de commande de bras articulé conçus pour commander le bras articulé 104 afin que ce dernier déplace la sonde 106 par rapport à l'objet 102.

L'objet 102 est par exemple une pièce mécanique que l'on souhaite examiner par contrôle non destructif ou bien, dans un contexte médical, une partie de corps humain que l'on souhaite contrôler de manière non invasive.

Dans le mode de réalisation de la figure 1, l'objet 102 est immergé dans un liquide, tel que de l'eau 110, et la sonde 106 est maintenue à distance de l'objet 102 afin que l'eau 110 les sépare.

La sonde 106 comporte tout d'abord un boîtier 112, c'est-à-dire un élément de structure indéformable qui sert de référentiel attaché à la sonde 106.

La sonde 106 comporte en outre N transducteurs $114_1$, ...,$114_N$ disposés linéairement dans le boîtier 112 et attachés à ce dernier. Les transducteurs $114_1$, ...,$114_N$ sont conçus pour émettre des ondes ultrasonores en direction de l'objet 102 en réponse à des signaux de commande identifiés sous la référence générale C, selon des directions principales parallèles les unes aux autres, indiquées par des flèches en pointillés sur la figure 1, et dans un plan principal qui est celui de la figure.

Les transducteurs $114_1$, ...,$114_N$ sont en outre conçus pour détecter des échos des ondes ultrasonores se réfléchissant sur et dans l'objet 102 et pour fournir des signaux de mesure identifiés sous la référence générale M et correspondant à ces échos.

Le dispositif de sondage 100 comporte en outre un circuit électronique 116 de commande des transducteurs $114_1$,...$114_N$ de la sonde 106 et de traitement des signaux de mesure M. Le circuit électronique 116 est connecté à la sonde 106 afin de lui transmettre les signaux de commande C et afin de recevoir les signaux de mesure M. Le circuit électronique 116 est par exemple celui d'un ordinateur. Le circuit électronique 116 présente une unité centrale de traitement 118, telle qu'un microprocesseur conçu pour émettre vers la sonde 106 les signaux de commande C et pour recevoir de la sonde 106 les signaux de mesure M, et une mémoire 120 dans laquelle est enregistré un programme d'ordinateur 122.

Le programme d'ordinateur 122 comporte une boucle d'instructions 124 à 138 pouvant être exécutée une ou plusieurs fois. Dans la suite de la description, un rang d'itération $p$ sera utilisé pour distinguer les différentes itérations de la boucle d'instructions 124 à 138. L'exécution initiale de la boucle correspond à $p$ égal à zéro (première itération), tandis que chaque répétition de la boucle correspond à la valeur de $p$ : $p$ est égal à un pour la première répétition (c'est-à-dire la deuxième itération), à deux pour la deuxième répétition (c'est-à-dire la troisième itération), etc.

Le programme d'ordinateur 122 comporte tout d'abord des instructions 124 conçues pour déterminer des retards d'émission $L^p = \left\{ L_1^p,...,L_N^p \right\}$, où $L_n^p$ est le retard d'émission à appliquer au transducteur $114_n$, à partir de retards initiaux $E^0 = \left\{ E_1^0,...,E_N^0 \right\}$ et, le cas échéant, de retards d'émission complémentaires $E^1 = \left\{ E_1^1,...,E_N^1 \right\},...,E^p = \left\{ E_1^p,...,E_N^p \right\}$ qui auront été déterminés par les instructions 136 décrites plus loin. Dans le mode de réalisation décrit, les retards d'émission $L^p$ sont déterminés en additionnant les retards initiaux $E^0$ et les retards d'émission complémentaires $E^1,...,E^p$ : $L^p = E^0 + E^1 + ... + E^p$. A la première exécution des instructions 124, c'est-à-dire lorsque $p$ est égal à zéro, les retards d'émission $L^0$ sont égaux aux retards initiaux $E^0$ : $L^0 = E^0$. Les retards initiaux $E^0$ sont prédéfinis dans le programme d'ordinateur 122. Il s'agit par exemple de retards nuls (aucun retard entre

les transducteurs $114_1$, ...,$114_N$), en particulier dans le cas où aucune information, même approximative, n'est connue sur la géométrie de l'objet 102. En variante, les retards initiaux $E^0$ peuvent être non nuls, et engendrent par exemple un front d'onde partiellement adapté à la géométrie de l'objet 102 en première approximation. Cette variante est par exemple utilisée dans le cas où la géométrie de l'objet 102 est déjà au moins partiellement connue.

Le programme d'ordinateur 122 comporte en outre des instructions 126 conçues pour commander les transducteurs $114_1$, ...,$114_N$ afin qu'ils émettent vers l'objet 102 des ondes ultrasonores ayant des retards d'émission $L^p$ les unes par rapport aux autres. A cet effet, les instructions 126 sont conçues pour transmettre à la p-ième itération des signaux de commande C notés $C^p = \left\{ C_1^p, ..., C_N^p \right\}$ aux transducteurs $114_1$, ...,$114_N$, où $C_n^p$ est la commande transmise au transducteur $114_n$ devant présenter un retard d'émission $L_n^p$. Ces signaux de commande $C^p$ sont conçus afin que les transducteurs $114_1$, ...,$114_N$ émettent chacun une onde ultrasonore impulsionnelle de pseudo-période temporelle $T$, les impulsions étant ainsi décalées dans le temps les unes par rapport aux autres des retards d'émission $L^p$. Les retards d'émission $L^p$ ont pour but de compenser les différences entre les distances séparant chaque transducteur de l'objet 102 pour le trajet aller, afin que les ondes ultrasonores émises par les transducteurs $114_1$, ...,$114_N$ atteignent au même instant l'objet 102.

Le programme d'ordinateur 122 comporte en outre des instructions 128 conçues pour recevoir à la p-ième itération, depuis les transducteurs, des signaux de mesure M notés $M^p = \left\{ M_1^p, ..., M_N^p \right\}$, où $M_n^p$ est le signal de mesure fourni par le transducteur $114_n$, mesurant en particulier les échos dus aux réflexions des ondes ultrasonores sur l'objet 102. Les instructions 128 sont en outre conçues pour enregistrer les signaux de mesure $M^p$. Dans le mode de réalisation décrit, les instructions 128 sont conçues pour enregistrer le signal de mesure $M_n^p$ de chaque transducteur $114_n$ sur une porte temporelle de durée prédéterminée et débutant, par exemple, lorsque le signal de commande $C_n^p$ de ce transducteur $114_n$ est émis. Les enregistrements des signaux de mesure $M^p$ sont notés $EN^p = \left\{ EN_1^p, ..., EN_N^p \right\}$, où $EN_n^p$ est l'enregistrement du signal $E_n^p$ du transducteur $114_n$. Les signaux de commande $C^p$ incluant les retards d'émission $L^p$, les enregistrements $EN^p$ des signaux de mesure $M^p$ intègrent eux aussi ces retards d'émission.

Le programme d'ordinateur 122 comporte en outre des instructions 130 conçues pour déterminer des décalages de réception $R^p = \left\{ R_1^p, ..., R_N^p \right\}$ des enregistrements $EN^p$, à partir des retards d'émission $L^p$, $R_n^p$ étant le décalage de réception de l'enregistrement $EN_n^p$. Dans le mode de réalisation décrit, les décalages de réception $R^p$ sont déterminés au moyen de la formule suivante : $R_n^p = \max\left( L_1^p, ..., L_N^p \right) - L_n^p$. Les décalages de réception $R^p$ ont pour but de compenser les différences entre les distances séparant chaque transducteur de l'objet pour le trajet retour, afin que les ondes ultrasonores, qui sont supposées se réfléchir au même instant sur la surface de l'objet 102 grâce aux retards d'émission $L^p$, soient synchronisées et donc considérées dans l'enregistrement comme atteignant au même instant les transducteurs $114_1$, ...,$114_N$.

Le programme d'ordinateur 122 comporte donc des instructions 132 conçues pour décaler les enregistrements $EN^p$ des signaux de mesure $M^p$ en fonction des décalages de réception $R^p$. Les enregistrements ainsi décalés sont notés $\underline{EN}^p = \left\{ \underline{EN}_1^p, ..., \underline{EN}_N^p \right\}$, où $\underline{EN}_n^p$ est l'enregistrement décalé du signal $M_n^p$ du transducteur $114_n$.

Le programme d'ordinateur 122 comporte en outre des instructions 134 conçues pour déterminer des temps de vol aller retour $t^p = \left\{ t_1^p, ..., t_N^p \right\}$, où $t_n^p$ est le temps de vol aller retour déterminé à partir de l'enregistrement décalé $\underline{EN}_n^p$ correspondant au transducteur $114_n$. Ainsi, les temps de vol aller retour $t^p$ tiennent compte des retards d'émission $L^p$ et des décalages de réception $R^p$. Dans le mode de réalisation décrit, le temps de vol aller retour $t_n^p$ pour chaque transducteur $114_n$ est déterminé en détectant, par exemple, le maximum de l'enveloppe du signal de mesure correspondant $M_n^p$, enregistré dans l'enregistrement décalé $\underline{EN}_n^p$.

Le programme d'ordinateur 122 comporte en outre des instructions 136 conçues pour déterminer de nouveaux retards d'émission complémentaires $E^{p+1}$ à partir des temps de vol aller retour $t^p$. Dans le mode de réalisation décrit, les retards d'émission complémentaires $E^{p+1}$ sont déterminés au moyen de la formule suivante :

$$E_n^{p+1} = \frac{1}{2}\left[\max\left(t_1^p,...,t_N^p\right) - t_n^p\right].$$

Le programme d'ordinateur 122 comporte en outre des instructions 138 conçues pour évaluer un test d'arrêt, afin de sortir de la boucle d'instructions 124 à 138 si le test d'arrêt est vérifié ou de poursuivre par une nouvelle itération dans le cas contraire. Dans ce dernier cas, les instructions 138 sont conçues pour revenir aux instructions 124 afin de provoquer une nouvelle itération de la boucle d'instructions 124 à 138, avec les nouveaux retards d'émission complémentaires $E^{p+1}$, de sorte que l'ensemble des retards d'émission complémentaires comprend les $p+1$ retards d'émission complémentaires $E^1,...,E^{p+1}$. Dans la présente description, c'est à ce moment que l'indice $p$ est incrémenté d'une unité, de sorte que l'ensemble des retards d'émission complémentaires est à ce moment noté $E^1,...,E^p$, en accord avec la description des instructions 124. Dans le mode de réalisation décrit, le test d'arrêt consiste à vérifier que l'inégalité suivante est vérifiée : $\max\left(E_1^p,...,E_N^p\right) \leq \dfrac{\lambda}{4v}$ , où $\lambda$ est la longueur d'onde dans l'eau à la fréquence centrale f de fonctionnement des transducteurs ($\lambda$ = v/f), où $v$ est la vitesse de propagation des ondes ultrasonores dans ce même milieu et où $E^p$ sont les derniers retards d'émission complémentaires déterminés par les instructions 136 (où ils étaient notés $E^{p+1}$). Concrètement, ce test signifie que, si la différence maximale entre les temps de vol aller retour $t^p$ déterminés par les instructions 134 est inférieure à $\lambda\big/4v$ , alors il peut être considéré en première approximation que ces temps de vol sont égaux et que la surface de l'objet 102 a bien été atteinte simultanément par toutes les ondes émises. En variante, les instructions 138 peuvent être conçues pour sortir de la boucle d'instructions 124 à 138 au bout d'un nombre prédéterminé d'exécutions de la boucle, par exemple quatre ou cinq, soit $p$ égal à trois ou quatre.

Si le résultat du test d'arrêt commande de sortir de la boucle d'instructions 124 à 138, alors les instructions 138 sont conçues pour passer à des instructions 140 d'analyse des derniers temps de vol aller retour calculés par les instructions 134. Ces derniers temps de vol aller retour sont alors notés $t^f$.

Le programme d'ordinateur 122 comporte donc ces instructions 140 conçues tout d'abord pour déterminer un temps de vol aller retour $Tc$ commun à tous les transducteurs, par exemple défini par $Tc = \left\langle t_n^f \right\rangle_{1 \leq n \leq N}$ , où $\langle \, \rangle$ désigne l'opération de moyenne. $Tc$ est la valeur de première approximation des temps de vol aller retour $t^f$ considérés comme tous égaux en sortie de boucle d'instructions 124 à 138.

De façon astucieuse, l'invention tire profit du fait que cette valeur commune de temps de vol aller retour $Tc$ pour l'ensemble des transducteurs intègre finalement les derniers retards d'émission appliqués aux transducteurs, notés $L^f$, et les derniers décalages de réception appliqués aux enregistrements, notés $R^f$. Conformément au calcul appliqué pour les décalages de réception, il est alors possible de retrouver le temps de vol spéculaire réel noté $t_n$ entre chaque transducteur 114$_n$ et la surface de l'objet 102, grâce à la relation suivante :

$$\forall n, \quad Tc = L_n^f + t_n - R_n^f .$$

On rappelle que le temps de vol spéculaire $t_n$ entre le centre du transducteur 114$_n$ et la surface de l'objet 102 est le temps minimal mis par un signal émis par ce transducteur pour revenir sous forme d'écho suite à une réflexion contre la surface de l'objet. Il est alors significatif de la plus courte distance séparant le transducteur 114$_n$ de la surface de l'objet 102, c'est-à-dire du segment de droite normal à cette surface et la reliant au transducteur 114$_n$ : ce segment sera qualifié dans la suite de trajet spéculaire.

Les instructions 140 sont alors également conçues pour déterminer, pour chaque transducteur 114$_n$, le temps de vol spéculaire $t_n$ qui lui est associé selon le calcul suivant :

$$t_n = Tc - L_n^f + R_n^f .$$

En variante, les derniers décalages de réception $R^f$ pourraient ne pas être appliqués aux derniers enregistrements, ce qui simplifierait l'équation précédente qui deviendrait alors : $t_n = Tc - L_n^f$ .

Enfin, les instructions 140 sont conçues pour déterminer, pour chaque transducteur 114$_n$, la distance $d_n$ qui le sépare de la surface de l'objet 102, selon la relation suivante :

$$d_n = \frac{1}{2} v \cdot t_n$$ , où $v$ est la vitesse de propagation des ondes ultrasonores dans l'eau.

Le programme d'ordinateur 122 comporte en outre des instructions 142 conçues pour calculer, dans un repère (O, x, y) lié au boîtier 112 de la sonde et définissant son plan de contrôle, les coordonnées $(x_n, y_n)$ des points $P_n$ de la surface de l'objet 102 se trouvant aux extrémités des trajets spéculaires associés aux transducteurs $114_n$. Ces points $P_n$ sont définis comme faisant partie de la surface de l'objet 102 (plus précisément de son intersection avec le plan de contrôle) et des cercles (en tant qu'intersections de sphères avec le plan de contrôle) respectivement centrés sur les transducteurs et de rayons $d_n$ : en d'autres termes, la surface de l'objet 102 telle que représentée dans le plan de contrôle de la sonde est naturellement définie comme étant une courbe tangentielle à l'ensemble des cercles respectifs de centres $C_n$ (i.e. les centres ponctuels de chaque transducteur $114_n$) et de rayons $d_n$, ou autrement dit comme l'enveloppe de ces cercles. Une méthode de résolution de ce problème purement géométrique est par exemple donnée dans le document de F. Assouline, intitulé « Migration profondeur et démigration pour l'analyse de vitesse de migration 3D », constituant une thèse présentée à l'Université de Pau et des Pays de l'Adour, Ecole doctorale des sciences exactes et de leurs applications, le 4 juillet 2001. Plus particulièrement au chapitre 1.3.4 intitulé « Migration 3D de Kirchhoff par déport commun », la détermination d'une enveloppe d'isochrones est donnée par un système d'équations 1.72 qui peut se simplifier au cas par cas selon le mode de réalisation choisi.

Ainsi par exemple, dans le cas bidimensionnel d'une sonde où les N transducteurs sont disposés linéairement dans le boîtier 112, les coordonnées de leurs centres peuvent s'exprimer sous la forme $(c_n, 0)$ dans le repère (O, x, y) et le système d'équation 1.72 appliqué aux points $P_n$ par les instructions 142 se simplifie de la façon suivante :

$$\forall n, \quad 1 \le n \le N-1, \quad \begin{cases} x_n = c_n - d_n \cdot d_n' \\ y_n = d_n\sqrt{1 - d_n'} \end{cases} \text{, où } d_n' = \frac{d_{n+1} - d_n}{c_{n+1} - c_n}.$$

On remarque qu'avec une sonde à N transducteurs, la surface à détecter est décrite par N-1 points $P_n$, le calcul des dérivées discrètes $d_n$ ne permettant pas de connaître le dernier point de la surface.

Le programme d'ordinateur 122 comporte en outre des instructions 144 conçues pour reconstituer géométriquement la surface à détecter par une interpolation linéaire. Cette approximation est valide pour des objets ne présentant pas de variations de surface trop brusques entre deux transducteurs successifs de la sonde, ce qui est vrai dans la plupart des applications où les surfaces contrôlées sont lentement variables. En variante, l'interpolation linéaire peut être remplacée par une interpolation polynomiale. Dans le cas d'une sonde à N transducteurs disposés linéairement, c'est un profil de la surface de l'objet 102 dans le plan de contrôle de la sonde qui est ainsi obtenu.

Mais les instructions 142 et 144 peuvent être aisément adaptées à une sonde matricielle dans laquelle les transducteurs sont disposés bidimensionnellement en matrice. Dans ce cas, le principe de reconstitution géométrique de la surface de l'objet 102 consiste plus généralement à calculer la surface tangentielle à une famille de sphères de rayons $d_i$ et dont les centres $C_i$ sont les centres des transducteurs de la matrice.

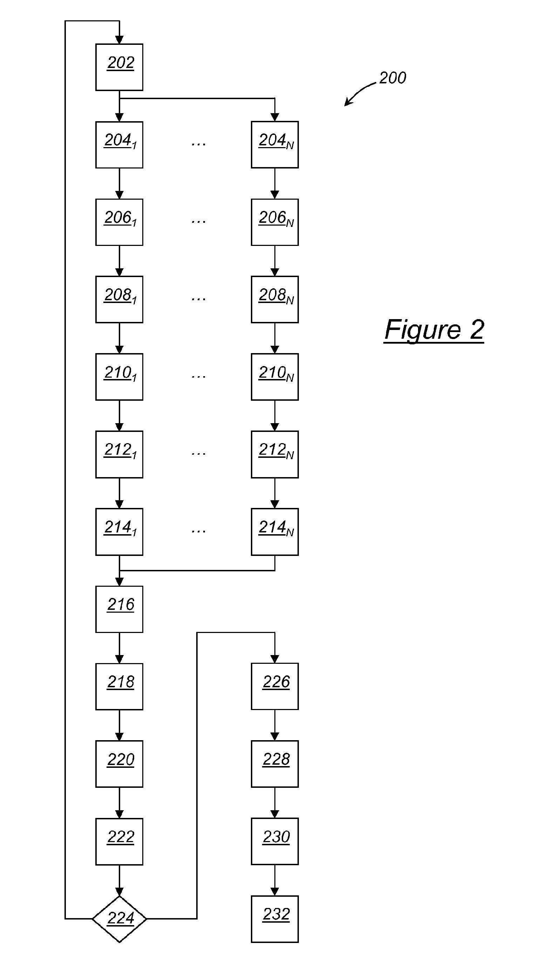

En référence à la figure 2, un procédé 200 de détermination d'une surface de l'objet 102 mis en oeuvre par le dispositif 100 de la figure 1 va à présent être décrit.

Au cours d'une étape 202, l'unité de traitement 118 exécutant les instructions 124 détermine les retards d'émission $L^p = \left\{ L_1^p, ..., L_N^p \right\}$ à partir des retards initiaux $E^0$ et, le cas échéant, des retards d'émission complémentaires $E^1, ..., E^p$ qui auront été déterminés à l'étape 222 décrite plus loin.

Au cours d'étapes $204_1$ à $204_N$, l'unité de traitement 118 exécutant les instructions 126 commande chaque transducteur $114_n$ afin qu'il émette des ondes ultrasonores vers l'objet 102, les ondes ultrasonores émises par les transducteurs $114_1, ..., 114_N$ ayant les retards d'émission $L^p$ les unes par rapport aux autres. A cet effet, l'unité de traitement 118 exécutant les instructions 126 transmet chaque signal de commande $C_n^p$ au transducteur $114_n$ correspondant, les signaux de commande $C^p$ incluant les retards d'émission $L^p$.

Au cours d'étapes $206_1$ à $206_N$, l'unité de traitement 118 exécutant les instructions 128 débute, suite à la transmission de chaque signal de commande $C_n^p$ vers le transducteur $114_n$ correspondant, l'enregistrement du signal de mesure $M_n^p$ fourni par ce transducteur $114_n$.

Au cours d'étapes $208_1$ à $208_N$, chaque transducteur $114_1, ..., 114_N$ émet, suite à la réception de son signal de commande, une onde ultrasonore impulsionnelle de fréquence centrale f. Ainsi, les impulsions sont décalées dans le temps les unes par rapport aux autres des retards d'émission $L^p$.

Au cours d'étapes $210_1$ à $210_N$, chaque transducteur $114_n$ reçoit les échos des ondes ultrasonores réfléchies sur et dans l'objet 102.

Au cours d'étapes $212_1$ à $212_N$, chaque transducteur $114_n$ fournit son signal de mesure $M_n^p$, mesurant en particulier les échos des ondes ultrasonores sur la surface de l'objet 102. L'unité de traitement 118 exécutant les instructions 128 reçoit ce signal $M_n^p$ et l'enregistre dans l'enregistrement $EN_n^p$.

Au cours d'étapes $214_1$ à $214_N$, l'unité de traitement 118 exécutant les instructions 128 stoppe l'enregistrement $EN_n^p$ du signal $M_n^p$ du transducteur $114_n$.

Au cours d'une étape 216, l'unité de traitement 118 exécutant les instructions 130 détermine les décalages de réception $R^p$ à partir des retards d'émission $L^p$.

Au cours d'une étape 218 l'unité de traitement 118 exécutant les instructions 132 décale les enregistrements $EN^p$ des signaux de mesure $M^p$ en fonction des décalages de réception $R^p$, afin d'obtenir les enregistrements décalés $EN^p$.

Au cours d'une étape 220, l'unité de traitement 118 exécutant les instructions 134 détermine les temps de vol aller retour $t^p = \left\{ t_1^p, ..., t_N^p \right\}$ entre les transducteurs $114_1...114_N$ et l'objet 102 tenant compte des retards d'émission $L^p$ et des décalages de réception $R^p$, à partir des enregistrements décalés $\underline{EN}^p$.

Au cours d'une étape 222, l'unité de traitement 118 exécutant les instructions 136 détermine de nouveaux retards d'émission complémentaires $E^{p+1}$ à partir des temps de vol aller retour $t^p$.

Ainsi, on remarquera que les étapes 216 à 222 permettent de déterminer des retards d'émission complémentaires $E^{p+1}$ à partir des signaux de mesure $M^p$.

Au cours d'une étape 224, l'unité de traitement 118 exécutant les instructions 138 détermine l'arrêt ou la poursuite de la boucle d'instructions 124 à 138, et, dans ce dernier cas, incrémente $p$ d'une unité avant de retourner à l'étape 202.

Si le test d'arrêt est vérifié, on passe à une étape 226 au cours de laquelle l'unité de traitement 118 exécute les instructions 140 pour déterminer le temps de vol spéculaire $t_n$ associé à chaque transducteur $114_n$.

Au cours d'une étape 228 suivante, l'unité de traitement 118 exécutant les instructions 140 détermine à partir de chaque temps de vol spéculaire $t_n$, chaque distance $d_n$ qui sépare chaque transducteur $114_n$ de la surface de l'objet 102.

Ensuite, au cours d'une étape 230, l'unité de traitement 118 exécutant les instructions 142 détermine N-1 points $P_1$, ..., $P_{N-1}$ de la surface de l'objet 102.

Enfin, au cours d'une étape 232, l'unité de traitement 118 exécutant les instructions 144 reconstitue géométriquement la surface de l'objet 102 par interpolation, en particulier par interpolation linéaire.

Les étapes 226 à 232 effectuent des traitements simples donc rapides qui n'ajoutent pas de complexité significative à l'ensemble du procédé de la figure 2. Ainsi, l'invention propose un procédé « temps réel » de détection de la surface, même complexe, d'un objet dont l'état de surface n'est pas connu au préalable. On peut donc qualifier ce procédé d'adaptatif permettant une imagerie en temps réel.

Le procédé de la figure 2, compatible avec un fonctionnement à haute cadence, est mis en oeuvre à chaque position du déplacement de la sonde 106 par rapport à l'objet 102. Dans le cas où l'objet présente de faibles variations de géométrie le long de ce déplacement, les retards d'émission initiaux $E^0$ à une position donnée sont avantageusement pris égaux aux derniers retards d'émission $L^p$ déterminés à une position précédente, notamment la position directement précédente. Ceci permet d'augmenter les vitesses d'inspection de pièces de surfaces très étendues par diminution du nombre de tirs à chaque position.

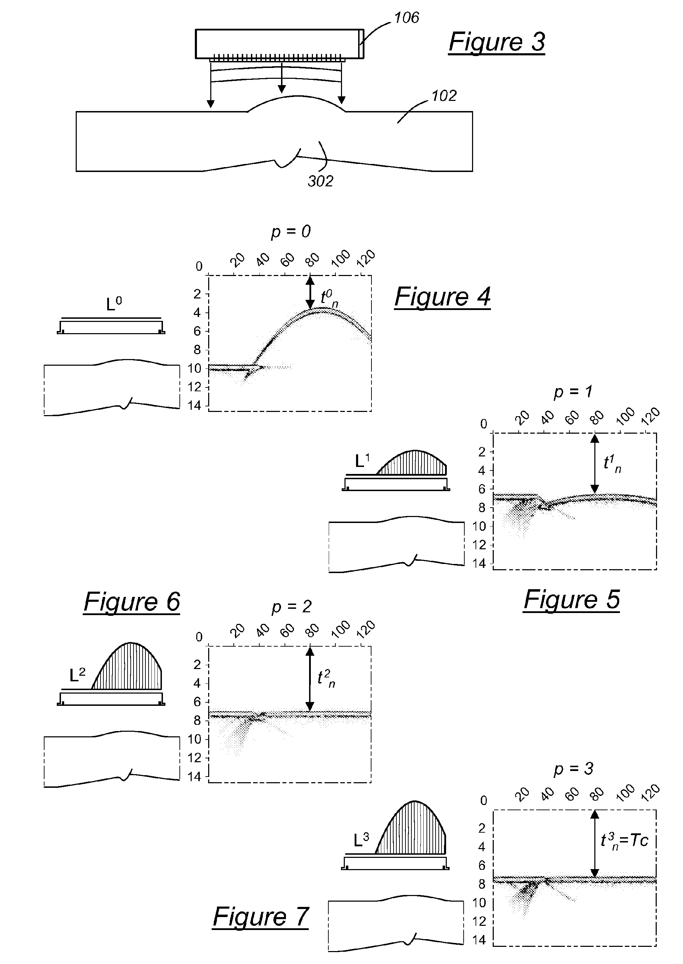

En référence aux figures 3 à 9, un premier exemple d'utilisation du procédé de détermination de surface de la figure 2 va être détaillé.

En référence à la figure 3, dans l'exemple décrit, l'objet 102 est une pièce métallique dont la géométrie complexe est représentative d'un bourrelet de soudure 302 que la sonde 106 du dispositif de sondage 100 est destinée à sonder. La sonde 106 comporte par exemple N = 128 transducteurs.

Afin de sonder le bourrelet de soudure 302, le procédé de la figure 2 est mis en oeuvre.

A une première exécution de la boucle d'étapes, c'est-à-dire lorsque $p$ est égal à zéro (première itération), des ondes ultrasonores sont émises aux étapes $208_1$ à $208_N$ par les transducteurs avec des retards d'émission $L^0$ égaux aux retards initiaux $E^0$ : $L^0 = E^0$, nuls dans l'exemple décrit.

Des enregistrements décalés $\underline{EN}^p$ sont ensuite obtenus à l'étape 218. En référence à la figure 4, l'amplitude de l'enveloppe des signaux décalés $\underline{EN}^p$ est déterminée à l'étape 220. Ils sont représentés sur la figure 4, dans laquelle l'axe vertical correspond au temps, l'axe horizontal aux transducteurs et l'amplitude de l'enveloppe au niveau de gris des points. Cette représentation est connue, comme indiqué précédemment, sous le nom de « B-scan ». Sur cette représentation B-scan, la distance aller retour $t_n^0$ pour chaque transducteur $114_n$ est déterminée comme étant la distance séparant l'origine des temps du maximum de l'amplitude d'enveloppe, c'est-à-dire du point le plus foncé sur la ligne verticale correspondant au transducteur $114_n$.

En variante, on notera que la représentation B-scan peut être modifiée en appliquant un traitement de moyenne glissante aux signaux de mesure enregistrés dans les enregistrements décalés $\underline{EN}^p$.

Les retards d'émission complémentaires $E^1$ sont alors déterminés à l'étape 222, et la poursuite de l'exécution du programme est décidée à l'étape 224 de sorte qu'une première répétition (deuxième itération) des étapes est réalisée ($p$ est incrémenté à 1).

La figure 5 représente le B-scan obtenu à l'étape 220 de cette première répétition (ou deuxième itération, p=1), à partir des retards d'émission $L^1 = E^0 + E^1$. Les retards d'émission complémentaires ayant été calculés à partir des temps de trajet aller retour, on s'attendrait à obtenir une surface d'objet sondé horizontale qui indiquerait que les ondes ultrasonores de tous les transducteurs atteignent au même instant le bourrelet de soudure 302. Cependant, on remarque que ce n'est pas le cas et que la ligne de surface, correspondant au bourrelet de soudure 302, est toujours un peu bombée.

En effet, les inventeurs ont déterminé que, pour des pièces de géométrie complexe, la première itération et sa répétition jusqu'à l'étape 220 ne suffisent pas pour corriger les écarts de géométrie et les phénomènes de superposition d'ondes sur un même signal de mesure qui en découlent. En effet, chaque transducteur détecte un écho qui est le produit de l'interférence entre l'écho résultant de sa propre émission d'ondes ultrasonores et les échos résultant des ondes ultrasonores émises par les transducteurs voisins. Néanmoins il est possible d'adapter l'appareil de sondage 100 à des géométries très complexes, telles que le bourrelet de soudure 302, en itérant plusieurs fois complètement les étapes du procédé 200 de la figure 2.

Ainsi, la figure 6 représente le B-scan obtenu à l'étape 220 de la deuxième répétition (ou troisième itération, p=2), à partir des retards d'émission $L^2 = E^0 + E^1 + E^2$, tandis que la figure 7 représente le B-scan obtenu à l'étape 220 de la troisième répétition (ou quatrième itération, p=3), à partir des retards d'émission $L^3 = E^0 + E^1 + E^2 + E^3$. On remarque que la surface de l'objet sondé est pratiquement horizontale sur cette dernière figure, indiquant que l'appareil de sondage 100 est adapté à la géométrie de l'objet 102, c'est-à-dire que le front d'onde ultrasonore formé par l'ensemble des ondes engendrées par tous les transducteurs $114_1...114_N$ a la même courbure que la surface du bourrelet de soudure 302. Lors de cette dernière étape, tous les temps de vol aller retour peuvent être considérés comme égaux à une même valeur Tc.

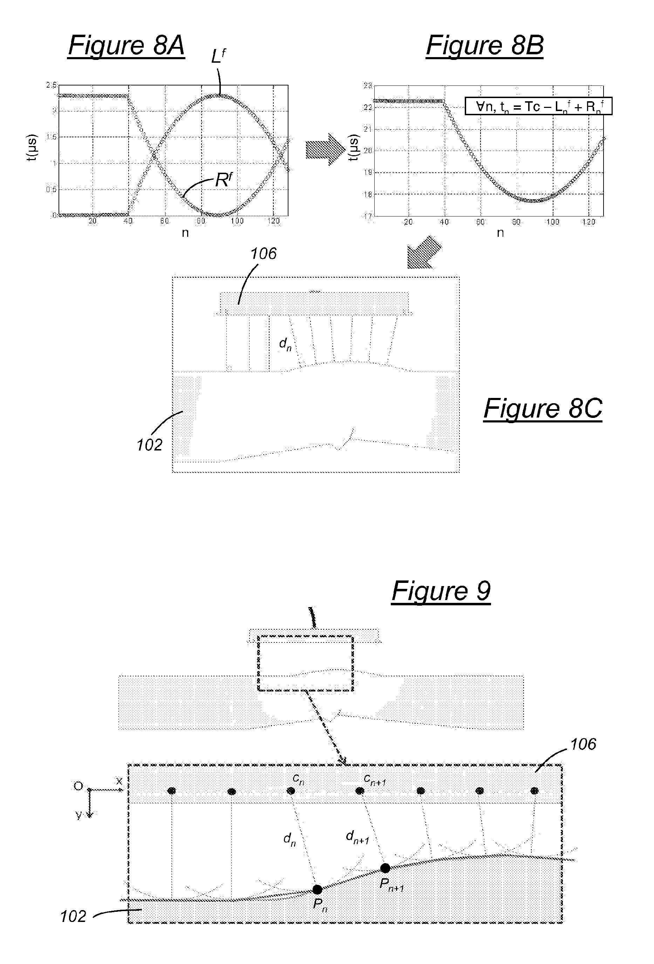

Les figures 8A, 8B et 8C illustrent le déroulement des étapes 226 et 228 sur exécution des instructions 140. Sur la figure 8A sont représentés les derniers retards d'émission $L^f$ appliqués aux N transducteurs et les derniers décalages de réception $R^f$ appliqués aux enregistrements. Sur la figure 8B sont représentés les temps de vol spéculaires résultants, calculés à l'étape 226, pour une valeur $Tc$ de 20 μs. Enfin, sur la figure 8C sont illustrés les distances $d_n$ qui séparent chaque transducteur $114_n$ de la surface de l'objet 102, c'est-à-dire les longueurs des trajets spéculaires telles que calculées à l'étape 228.

La figure 9 illustre le résultat des étapes 230 et 232 sur exécution des instructions 142 et 144. La surface de l'objet 102 est reconstituée géométriquement par interpolation linéaire à partir des points $P_n$ eux mêmes déterminés à partir des positions $c_n$ des transducteurs $114_n$ et des distances $d_n$.

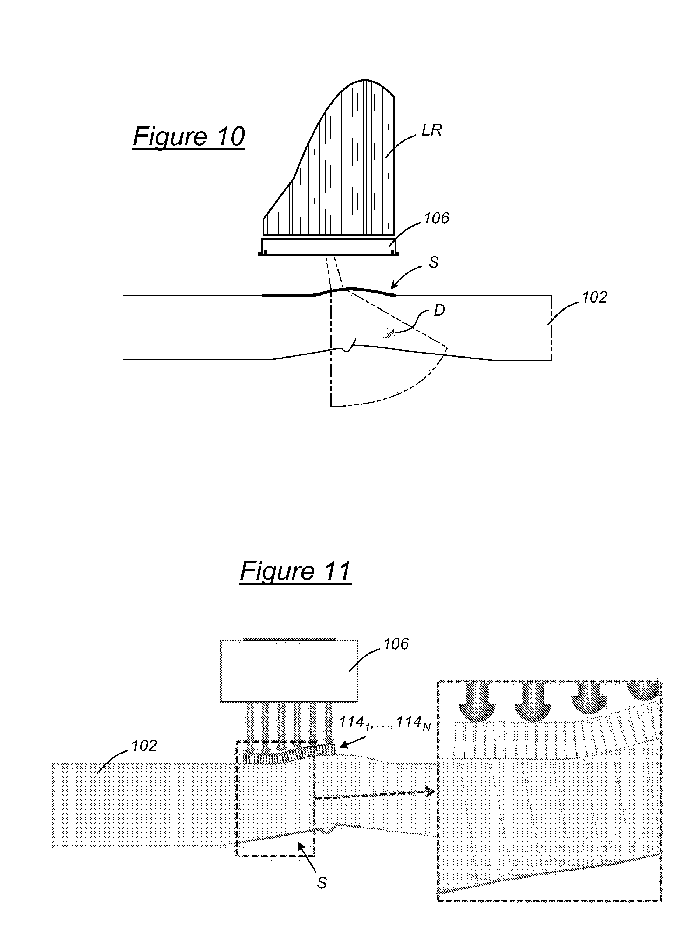

En reprenant l'exemple des figures 3 à 9, dans lequel l'objet 102 est une pièce métallique dont la géométrie complexe est représentative d'un bourrelet de soudure 302, deux applications différentes sont envisagées et illustrées sur les figures 10 et 11.

La première application du procédé précédemment détaillé est l'imagerie échographique de pièces métalliques en temps réel par focalisation en différents points. Cette application est illustrée sur la figure 10 avec une image de type S-scan obtenue en focalisant selon différents angles et à profondeur constante. Cette technologie repose sur une maîtrise à chaque instant de la profondeur et de l'angle de focalisation du faisceau ultrasonore par la définition d'un ensemble de lois de retards (une loi de retards pour chaque point de focalisation) évoluant dans le temps et appliqué à l'ensemble des transducteurs de la sonde. Mais, dans l'état de la technique, cette technologie nécessite une parfaite connaissance de la géométrie de l'objet sondé pour pouvoir adapter les lois de retards en conséquence. Ainsi, pour un objet de géométrie variable, le contrôle nécessiterait de reconstruire la surface de l'objet pour chaque position de la sonde afin d'adapter les lois de retards à la géométrie rencontrée et maîtriser un faisceau constant tout le long du déplacement et quel que soit l'état de surface de l'objet sondé.

Une solution, apportée par l'application du procédé de la figure 2, est illustrée sur la figure 10. Comme cela est visible, après reconstitution de la surface S de l'objet 102 grâce à l'application du procédé de la figure 2, un jeu de lois de retards LR est calculé en fonction de cette surface reconstituée pour focaliser selon différents angles et à profondeur constante. Un S-scan est obtenu, permettant notamment la détection de défauts éventuels D. L'utilisation de ce procédé permet ainsi de reconstituer la surface de l'objet à chaque position de la sonde et d'adapter en conséquence les lois de retards pour maîtriser une image de type S-scan en temps-réel en cours de déplacement.

La technologie classique utilisée pour l'obtention d'une image à partir d'un jeu de lois de retards adaptées grâce à l'application du procédé de détection selon l'invention est par exemple l'une de celles décrites dans les documents suivants :

- l'article de A. Lamarre et al, intitulé « Dynamic focusing of phased arrays for nondestructive testing: characterization and application », publié dans le e-Journal of Nondestructive Testing and Ultrasonics, n° 9, vol. 4, septembre 1999,
- l'article de J. Liang et al, intitulé « Ultrasonic Inspection of thick parts with phased array dynamic focusing », publié suite à la 10e European Conférence on Non-Destructive Testing, 7-11 juin 2010, Moscou,
- l'article de C. Holmes et al, intitulé « Post-processing of the full matrix of ultrasonic transmit-receive array data for non-destructive évaluation », publié dans NDT & E International, vol. 38, n° 8, pages 701-711, décembre 2005, et
- l'article de A. Fidahoussen et al, intitulé « Imaging of defects in several complex configurations by simulation-helped processing of ultrasonic array data », publié dans 36th Annual Review of Progress in Quantitative Non-Destructive Evaluation, vol. 29, pages 847-854, 2010.

La deuxième application du procédé précédemment détaillé, illustrée sur la figure 11, concerne une famille d'applications selon lesquelles les images échographiques s'obtiennent en appliquant des lois de retards permettant de focaliser dans la pièce après réflexion sur le fond. Ceci nécessite une connaissance préalable de la géométrie de la surface mais aussi du fond de l'objet à inspecter, comme par exemple évoqué dans l'article de A. Bazulin et al, intitulé « Algorithms and software development for welds automated ultrasonic inspection basing on phased arrays », publié suite à la 10e European Conférence on Non-Destructive Testing, 7-11 juin 2010, Moscou, ou dans l'article de A. Fidahoussen et al précédemment cité. Si la sonde à transducteurs multiples est destinée à être au contact de l'objet sondé 102, seule la surface du fond de l'objet 102 nécessite d'être reconstituée. Par une application stricte du procédé de la figure 2, cette surface du fond peut être géométriquement reconstituée relativement à la surface qui est au contact des transducteurs, l'unique différence résidant dans la vitesse des ondes ultrasonores qui, au contact, devient celle des ondes longitudinales se propageant dans le matériau de l'objet sondé.

La figure 11 illustre en particulier le cas où la sonde à transducteurs multiples est au contact de l'objet en utilisant la technologie décrite dans le brevet français publié sous le numéro FR 2 786 651. Dans ce document, les transducteurs sont attachés de manière mobile au boîtier afin de pouvoir épouser la géométrie de la surface de l'objet à sonder. Il est alors prévu des moyens pour déterminer les positions des transducteurs, et ainsi la géométrie de la surface de l'objet. L'application stricte du procédé de la figure 2 permet alors, à partir de la connaissance de la surface, d'en déduire la géométrie du fond de pièce.

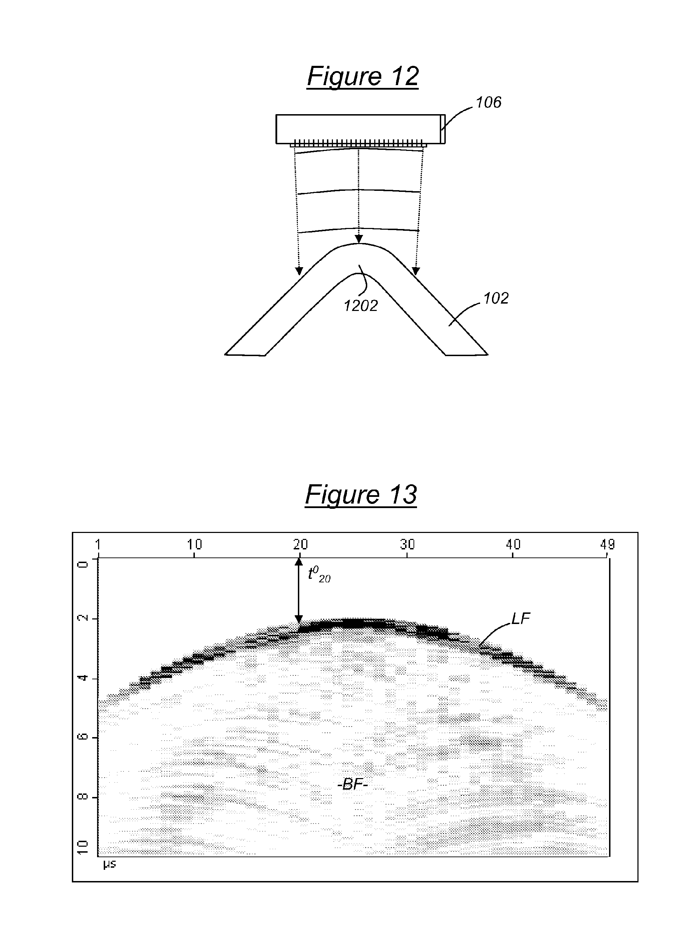

En référence aux figures 12 à 16, un second exemple d'utilisation du procédé de détermination de surface de la figure 2 va maintenant être détaillé. Ce second exemple concerne l'inspection de pièces composites.

En référence à la figure 12, dans l'exemple décrit, l'objet, référencé 102', est une pièce présentant un rayon de composite 1202 d'angle élevé que la sonde 106 du dispositif de sondage 100 est destinée à sonder. La sonde 106 comporte par exemple N = 49 transducteurs.

Afin de sonder le rayon de composite 1202, le procédé de la figure 2 est mis en oeuvre.

A une première exécution de la boucle d'étapes, c'est-à-dire lorsque $p$ est égal à zéro (première itération), des ondes ultrasonores sont émises aux étapes $208_1$ à $208_N$ par les transducteurs avec des retards d'émission $L^0$ égaux aux retards initiaux $E^0$ : $L^0 = E^0$, nuls dans l'exemple décrit.

Des enregistrements décalés $\underline{EN}^p$ sont ensuite obtenus à l'étape 218. En référence à la figure 13, l'amplitude de l'enveloppe des signaux décalés $\underline{EN}^p$ est déterminée à l'étape 220 et reproduite dans le B-scan correspondant. On remarque l'apparition d'une ligne de surface foncée LF, correspondant à l'écho de surface du rayon de composite 1202, suivie d'un important bruit de structure BF ne permettant en aucun cas de révéler la présence d'un éventuel défaut dans l'objet 102'.

Les retards d'émission complémentaires $E^1$ sont alors déterminés à l'étape 222, et la poursuite de l'exécution du programme est décidée à l'étape 224 de sorte qu'une première répétition (deuxième itération) des étapes est réalisée ($p$ est incrémenté à 1).

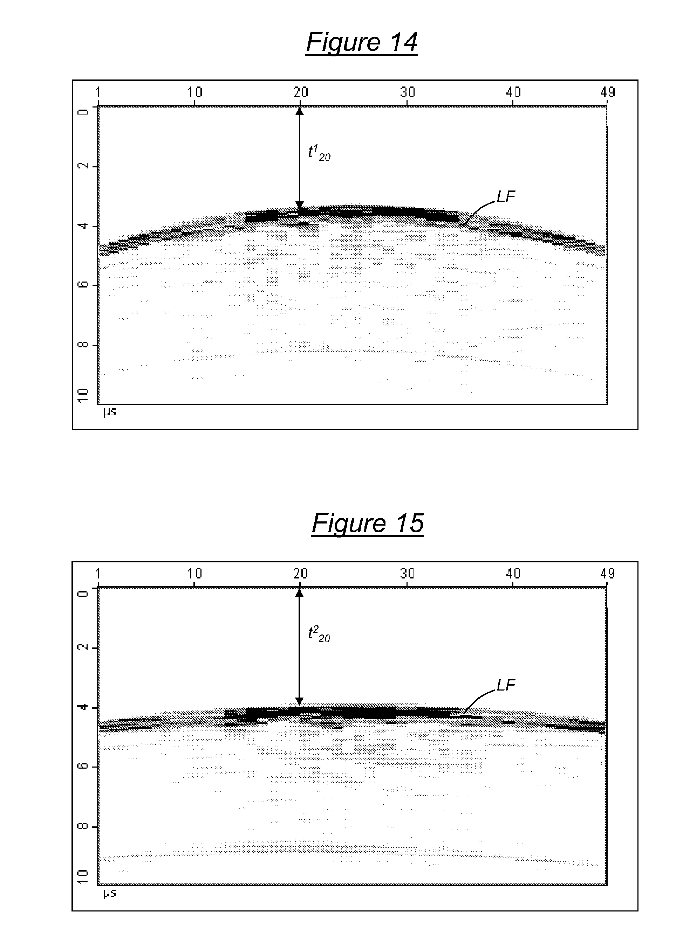

La figure 14 représente le B-scan obtenu à l'étape 220 de cette première répétition (ou deuxième itération, p=1), à partir des retards d'émission $L^1 = E^0 + E^1$. Là encore, la ligne de surface LF de l'objet sondé, correspondant à la surface du rayon de composite 1202, est toujours un peu bombée.

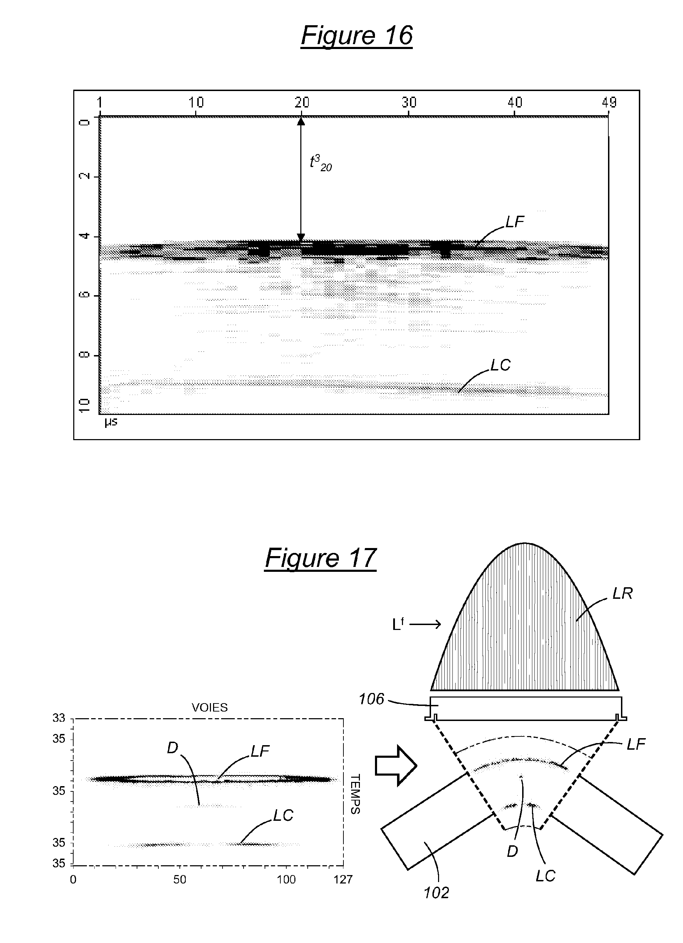

La figure 15 représente le B-scan obtenu à l'étape 220 de la deuxième répétition (ou troisième itération, p=2), à partir des retards d'émission $L^2 = E^0 + E^1 + E^2$, tandis que la figure 16 représente le B-scan obtenu à l'étape 220 de la troisième répétition (ou quatrième itération, p=3), à partir des retards d'émission $L^3 = E^0 + E^1 + E^2 + E^3$.

On remarque que la ligne foncée LF est pratiquement horizontale sur cette dernière figure, indiquant que le front d'onde ultrasonore formé par l'ensemble des ondes engendrées par tous les transducteurs $114_1...114_N$ a la même courbure que la surface du rayon de composite 1202. En outre, le bruit interne a disparu, laissant apparaître la structure réelle de l'objet 102', en particulier une ligne claire LC indiquant le fond de l'objet. Ainsi, tout défaut dans l'objet 102' devient apparent grâce à une répétition multiple de la boucle d'étapes précédemment décrite. En particulier, les défauts de type délaminage sont mieux détectés.

L'exécution des étapes 226 à 232 sur le résultat obtenu à la figure 16 permet alors de reconstituer géométriquement la surface courbe de l'objet 102'.

En reprenant l'exemple des figures 12 à 16, dans lequel l'objet 102' est une pièce composite présentant un rayon de composite 1202 d'angle élevé, une troisième application est envisagée et illustrée sur la figure 17.

Cette troisième application consiste à reconstruire une image échographique à partir du B-scan de la figure 16. Ce B-scan est représenté en partie gauche de la figure 17 avec un défaut D détecté dans l'objet 102'. La partie de droite est une image échographique dans laquelle les pixels du B-scan de gauche sont repositionnés dans le repère (O, x, y) grâce à la connaissance de la géométrie de la surface détectée et par comparaison avec sa forme linéaire dans le B-scan. Grâce à cette transformation, non seulement le défaut D est détecté, mais en outre il peut être localisé et mesuré avec précision dans l'image échographique reconstruite.

En outre, par déplacement de la sonde le long de l'objet, plusieurs images échographiques peuvent être reconstruites et concaténées en une seule image complète de l'objet.

Il apparaît clairement qu'un procédé et un dispositif tels que ceux décrits précédemment permettent de reconstituer finement la géométrie, bidimensionnelle ou respectivement tridimensionnelle, d'une surface d'objet détectée par échographie, cette reconstitution fine ouvrant la voie pour améliorer une pluralité d'applications.

On notera en outre que le procédé décrit précédemment permet une détection en temps réel et à niveau de qualité élevé.

On notera par ailleurs que l'invention n'est pas limitée aux modes de réalisation décrits précédemment. Il apparaîtra en effet à l'homme de l'art que diverses modifications peuvent être apportées aux modes de réalisation décrits ci-dessus, à la lumière de l'enseignement qui vient de lui être divulgué.

En particulier, les instructions de programme d'ordinateur pourraient être remplacées par des circuits électroniques dédiés aux fonctions réalisées lors de l'exécution de ces instructions.

En outre, le procédé selon l'invention peut être mis en oeuvre avec un déplacement mécanique de la sonde ou bien en procédant à un déplacement électronique d'une sous ouverture le long de l'ouverture totale du capteur où sont situés les transducteurs.

Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

## Revendications

1. Procédé de reconstruction de la géométrie d'une surface (S ; LF) d'un objet (102 ; 102') par sondage échographique à l'aide d'une sonde à ultrasons (106) munie d'une pluralité de transducteurs ($114_1$, ..., $114_N$), comprenant les étapes suivantes :

   - commander ($204_1$, ..., $204_N$) les transducteurs ($114_1$, ..., $114_N$) afin qu'ils émettent vers ladite surface (S ; LF) de l'objet des ondes ultrasonores présentant des retards d'émission initiaux ($L^0$) les unes par rapport aux autres,
   - exécuter au moins une fois la boucle d'étapes suivante, de manière à obtenir après au moins une itération un front d'onde reçu simultanément sur la surface :

      • recevoir ($212_1$, ..., $212_N$) depuis les transducteurs ($114_1$, ..., $114_N$) des signaux de mesure intermédiaires (M), mesurant en particulier des échos dus à des réflexions des ondes ultrasonores sur ladite surface (S ; LF) de l'objet,
      • corriger (202) les retards d'émission des transducteurs ($114_1$, ..., $114_N$) à l'aide des signaux de mesure intermédiaires (M) et commander ($204_1$, ..., $204_N$) les transducteurs ($114_1$, ..., $114_N$) afin qu'ils émettent vers ladite surface (S ; LF) de l'objet des ondes ultrasonores présentant les retards d'émission corrigés ($L^p$) les unes par rapport aux autres,

   - recevoir ($212_1$, ..., $212_N$) depuis les transducteurs ($114_1$, ..., $114_N$) des signaux de mesure finaux (M) résultant de la réflexion d'un front d'onde reçu simultanément sur ladite surface (S ; LF) de l'objet,

   **caractérisé en ce qu'**il comporte en outre les étapes suivantes :

   - détermination (226) de temps de vol, dits spéculaires et correspondant chacun au temps minimal mis par un signal émis par un transducteur pour revenir à ce transducteur sous forme d'écho suite à une réflexion contre la surface de l'objet, entre chaque transducteur ($114_1$, ..., $114_N$) et ladite surface (S ; LF) de l'objet à partir des signaux de mesure finaux (M) et des retards d'émission corrigés ($L^f$), et

- reconstitution géométrique (228, 230, 232) de ladite surface (S ; LF) de l'objet à partir des temps de vol spéculaires déterminés.

2. Procédé de reconstruction d'une géométrie de surface (S ; LF) selon la revendication 1, dans lequel la détermination (226) des temps de vol spéculaires comporte :

- la détermination d'un temps de vol aller retour, commun à tous les transducteurs ($114_1$, ..., $114_N$), du front d'onde reçu simultanément sur ladite surface (S ; LF) de l'objet, et
- le calcul des temps de vol spéculaires à partir de ce temps de vol aller retour, des retards d'émission corrigés ($L^f$) et de décalages de réception ($R^f$) appliqués aux transducteurs.

3. Procédé de reconstruction d'une géométrie de surface (S ; LF) selon la revendication 1 ou 2, dans lequel la reconstitution géométrique (228, 230, 232) de ladite surface (S ; LF) comporte :

- la détermination (228) de distances ($d_n$) séparant chaque transducteur ($114_1$, ..., $114_N$) de ladite surface (S ; LF) à partir des temps de vol spéculaires déterminés,
- le calcul (230) de coordonnées de points ($P_n$) de ladite surface (S ; LF) en fonction de coordonnées ($c_n$) des transducteurs ($114_1$, ..., $114_N$) et des distances déterminées ($d_n$), et
- la reconstitution géométrique (232) de ladite surface (S ; LF) par interpolation entre ces points ($P_n$).

4. Procédé de reconstruction d'une géométrie de surface (S ; LF) selon la revendication 3, dans lequel le calcul (230) des coordonnées de points ($P_n$) de ladite surface (S ; LF) est basé sur l'hypothèse que ladite surface est tangentielle à un ensemble de sphères centrées respectivement sur les transducteurs ($114_1$, ..., $114_N$) et de rayons respectifs correspondant aux distances déterminées ($d_n$).

5. Procédé de reconstruction d'une géométrie de surface (S ; LF) selon la revendication 3 ou 4, dans lequel les transducteurs ($114_1$, ..., $114_N$) sont disposés linéairement et la reconstitution géométrique (228, 230, 232) de ladite surface (S ; LF) comporte la reconstruction d'un profil de cette surface dans le plan de contrôle de la sonde.

6. Procédé de reconstruction d'une géométrie de surface (S ; LF) selon la revendication 5, dans lequel N transducteurs ($114_1$, ..., $114_N$) sont disposés linéairement, les coordonnées de leurs centres pouvant s'exprimer sous la forme ($c_n$, 0) dans un repère (O, x, y) lié à la sonde (106), et dans lequel les coordonnées ($x_n$, $y_n$) desdits points ($P_n$) de ladite surface (S ; LF) sont calculées à l'aide de la relation suivante :

$$\forall n,\ 1 \leq n \leq N\text{-}1,\ \begin{cases} x_n = c_n - d_n \cdot d_n{'} \\ y_n = d_n \sqrt{1 - d_n{'}} \end{cases},\ \text{où}\ d_n{'} = \frac{d_{n+1} - d_n}{c_{n+1} - c_n}\ \text{et}\ d_n\ \text{désigne la distance séparant le n-ième trans-}$$

ducteur de ladite surface.

7. Procédé de reconstruction d'une géométrie de surface (S ; LF) selon la revendication 3 ou 4, dans lequel les transducteurs ($114_1$, ..., $114_N$) sont disposés bidimensionnellement en matrice et la reconstitution géométrique (228, 230, 232) de ladite surface (S ; LF) comporte une reconstitution tridimensionnelle de cette surface.

8. Procédé de reconstruction d'une géométrie de surface (S ; LF) selon l'une quelconque des revendications 3 à 7, dans lequel la reconstitution géométrique (228, 230, 232) de ladite surface (S ; LF) se fait par interpolation linéaire ou bilinéaire entre lesdits points ($P_n$).

9. Programme d'ordinateur (122) téléchargeable depuis un réseau de communication et/ou enregistré sur un support (120) lisible par ordinateur et/ou exécutable par un processeur (118), **caractérisé en ce qu'**il comprend des instructions pour l'exécution des étapes d'un procédé de reconstruction de la géométrie d'une surface d'un objet (102 ; 102') par sondage échographique selon l'une quelconque des revendications 1 à 8, lorsque ledit programme est exécuté sur un ordinateur.

10. Dispositif de sondage à ultrasons comportant une sonde (106) comprenant un boîtier (112) et une pluralité de transducteurs ($114_1$, ..., $114_N$) à ultrasons attachés au boîtier (112), et des moyens de commande et de traitement (116) conçus pour :

- commander les transducteurs ($114_1$, ..., $114_N$) afin qu'ils émettent vers ladite surface (S ; LF) de l'objet des ondes ultrasonores présentant des retards d'émission initiaux ($L^0$) les unes par rapport aux autres,
- exécuter au moins une fois la boucle d'étapes suivante, de manière à obtenir après au moins une itération un front d'onde reçu simultanément sur la surface :

   • recevoir depuis les transducteurs ($114_1$, ..., $114_N$) des signaux de mesure intermédiaires (M), mesurant en particulier des échos dus à des réflexions des ondes ultrasonores sur ladite surface (S ; LF) de l'objet,
   • corriger les retards d'émission des transducteurs ($114_1$, ..., $114_N$) à l'aide des signaux de mesure intermédiaires (M) et commander les transducteurs ($114_1$, ..., $114_N$) afin qu'ils émettent vers ladite surface (S ; LF) de l'objet des ondes ultrasonores présentant les retards d'émission corrigés ($L^p$) les unes par rapport aux autres,

- recevoir depuis les transducteurs ($114_1$, ..., $114_N$) des signaux de mesure finaux (M) résultant de la réflexion d'un front d'onde reçu simultanément sur ladite surface (S ; LF) de l'objet,

**caractérisé en ce que** les moyens de commande et de traitement (116) comportent en outre :

- des moyens de détermination de temps de vol, dits spéculaires et correspondant chacun au temps minimal mis par un signal émis par un transducteur pour revenir à ce transducteur sous forme d'écho suite à une réflexion contre la surface de l'objet, entre chaque transducteur ($114_1$, ..., $114_N$) et ladite surface (S ; LF) de l'objet à partir des signaux de mesure finaux (M) et des retards d'émission corrigés ($L^f$), et
- des moyens de reconstitution géométrique de ladite surface (S ; LF) de l'objet à partir des temps de vol spéculaires déterminés.


## Patentansprüche

1. Verfahren zur Rekonstruktion der Geometrie einer Oberfläche (S; LF) eines Objekts (102; 102') durch Echoloten mit einer Ultraschallsonde (106), die mit mehreren Schallgebern ($114_1$, ..., $114_N$) versehen ist, das die folgenden Schritte umfasst:

- Steuern ($204_1$, ..., $204_N$) der Schallgeber ($114_1$, ..., $114_N$), damit sie zu der Oberfläche (S; LF) des Objekts Ultraschallwellen senden, die anfängliche Sendeverzögerungen ($L^0$) zueinander aufweisen,
- Ausführen mindestens einer darauf folgenden Schleife von Schritten derart, dass nach mindestens einer Iteration eine Wellenfront, die gleichzeitig auf der Oberfläche empfangen wird, erhalten wird:

   -- Empfangen ($212_1$,..., $212_N$) von den Schallgebern ($114_1$, ..., $114_N$) der Zwischenmesssignale (M), die insbesondere Echos aufgrund von Reflexionen der Ultraschallwellen auf der Oberfläche (S; LF) des Objekts zurückzuführen sind,
   -- Korrigieren (202) der Sendeverzögerungen der Schallgeber ($114_1$, ..., $114_N$) mit Hilfe der Zwischenmesssignale (M) und Steuern ($204_1$, ..., $204_N$) der Schallgeber ($114_1$, ..., $114_N$), damit sie zu der Oberfläche (S; LF) des Objekts Ultraschallwellen senden, die zueinander korrigierte Sendeverzögerungen ($L^p$) aufweisen,

- Empfangen ($212_1$,... $212_N$) von den Schallgebern ($114_1$, ..., $114_N$) der abschließenden Messsignale (M), die aus der Reflexion einer Wellenfront resultieren, die gleichzeitig auf der Oberfläche (S; LF) des Objekts empfangen wird,

**dadurch gekennzeichnet, dass** es außerdem die folgenden Schritte umfasst:

- Bestimmen (226) der Flugzeiten, Spiegelungsflugzeiten genannt, und die jeweils der Mindestzeit entsprechen, die ein von dem Schallgeber gesendetes Signal braucht, um zu diesem Schallgeber in Form eines Echos im Anschluss an eine Reflexion an der Oberfläche des Objekts zurückzukehren, zwischen jedem Schallgeber ($114_1$, ..., $114_N$) und der Oberfläche (S; LF) des Objekts ausgehend von den abschließenden Messsignalen (M) und den korrigierten Sendeverzögerungen ($L^f$), und
- geometrische Rekonstution (228, 230, 232) der Oberfläche (S; LF) des Objekts ausgehend von den bestimmten Spiegelungsflugzeiten.

2. Verfahren zur Rekonstruktion einer Oberflächengeometrie (S; LF) nach Anspruch 1, wobei das Bestimmen (226) der Spiegelungsflugzeiten Folgendes umfasst:

- Bestimmen einer Hin- Rückflugzeit, die alle Schallgeber ($114_1$, ..., $114_N$) gemeinsam haben, der Wellenfront, die gleichzeitig auf der Oberfläche (S; LF) des Objekts empfangen wird, und
- Berechnung der Spiegelungsflugzeiten ausgehend von dieser Hin- und Rückflugzeit der korrigierten Sendeverzögerungen ($L^f$) und der an die Schallgeber angewandten Empfangsversätze ($R^f$).

3. Verfahren zur Rekonstruktion einer Oberflächengeometrie (S; LF) nach Anspruch 1 oder 2, wobei die geometrische Rekonstitution (228, 230, 232) der Oberfläche (S; LF) Folgendes umfasst:

- Bestimmen (228) von Entfernungen ($d_n$), die jeden Schallgeber ($114_1$, ..., $114_N$) von der Oberfläche (S; LF) trennen, ausgehend von den bestimmten Spiegelungsflugzeiten,
- Berechnung (230) von Koordinaten von Punkten ($P_n$) der Oberfläche (S; LF) in Abhängigkeit von Koordinaten ($c_n$) der Schallgeber ($114_1$, ..., $114_N$) und den bestimmten Entfernungen ($d_n$), und
- geometrische Rekonstitution (233) der Oberfläche (S; LF) durch Interpolation zwischen diesen Punkten ($P_n$).

4. Verfahren zur Rekonstruktion einer Oberflächengeometrie (S; LF) nach Anspruch 3, wobei die Berechnung (230) der Koordinaten von Punkten ($P_n$) der Oberfläche (S; LF) auf der Annahme basiert, dass die Oberfläche zu einer Einheit von Kugeln tangential ist, die jeweils auf den Schallgebern ($114_1$, ..., $114_N$) zentriert sind, und die jeweilige Strahlen, die den bestimmten Entfernungen ($d_n$) entsprechen, haben.

5. Verfahren zur Rekonstruktion einer Oberflächengeometrie (S; LF) nach Anspruch 3 oder 4, wobei die Schallgeber ($114_1$, ..., $114_N$) linear angeordnet sind, und die geometrische Rekonstitution (228, 230, 232) der Oberfläche (S; LF) die Rekonstitution eines Profils dieser Oberfläche in der Prüfebene der Sonde umfasst.

6. Verfahren zur Rekonstruktion einer Oberflächengeometrie (S; LF) nach Anspruch 5, wobei N Schallgeber ($114_1$, ..., $114_N$) linear angeordnet sind, wobei die Koordinaten ihrer Mitten in der Form ($c_n$, 0) in einer Markierung (O, x, y), die mit der Sonde (106) verbunden ist, ausgedrückt werden kann, und wobei die Koordinaten ($x_n$, $y_n$) der Punkte ($P_n$) der Oberfläche (S; LF) anhand der folgenden Gleichung berechnet werden:

$$\forall n, 1 \leq N-1, \begin{cases} x_n = c_n - d_n \cdot d_n{}' \\ y_n = d_n \sqrt{1 - d_n{}'} \end{cases}, \text{ wobei } d_n{}' = \frac{d_{n+1} - d_n}{c_{n+1} - c_n}$$ und $d_n$ die Entfernung bezeichnet, die den n-ten Schallgeber von der Oberfläche trennt.

7. Verfahren zur Rekonstruktion einer Oberflächengeometrie (S; LF) nach Anspruch 3 oder 4, wobei die Schallgeber ($114_1$, ..., $114_N$) zweidimensional als Matrix angeordnet sind und die geometrische Rekonstitution (228, 230, 232) der Oberfläche (S; LF) eine dreidimensionale Rekonstitution dieser Oberfläche umfasst.

8. Verfahren zur Rekonstruktion einer Oberflächengeometrie (S; LF) nach einem der Ansprüche 3 bis 7, wobei die geometrische Rekonstitution (228, 230, 232) der Oberfläche (S; LF) durch lineare oder bilineare Interpolation zwischen den Punkten ($P_n$) erfolgt.

9. Computerprogramm (122), das von einem Kommunikationsnetz downloadbar und/oder auf einem von einem Computer lesbaren Medium (120) aufgezeichnet ist und/oder von einem Prozessor (118) ausgeführt werden kann, **dadurch gekennzeichnet, dass** es Anweisungen zur Ausführung der Schritte eines Verfahrens zur Rekonstruktion der Geometrie einer Oberfläche eines Objekts (102; 102') durch Echoloten nach einem der Ansprüche 1 bis 8 umfasst, wenn das Programm auf einem Computer ausgeführt wird.

10. Echolotvorrichtung, die eine Sonde (106) umfasst, die ein Gehäuse (112) und mehrere Schallgeber ($114_1$, ..., $114_N$) mit Ultraschall umfasst, die an dem Gehäuse (112) befestigt sind, und Mittel zum Steuern und Verarbeiten (116), die konzipiert sind, um:

- die Schallgeber ($114_1$, ..., $114_N$) zu steuern, damit sie zu der Oberfläche (S; LF) des Objekts Ultraschallwellen senden, die anfängliche Sendeverzögerungen ($L^0$) zueinander aufweisen,
- mindestens einmal die folgende Schleife von Schritten derart auszuführen, dass nach mindestens einer Iteration eine Wellenfront erhalten wird, die gleichzeitig auf der Oberfläche empfangen wird:

-- Empfangen von den Schallgebern ($114_1$, ..., $114_N$) der Signale von zwischen Messungen (M), die insbesondere Echos messen, die auf Reflexionen der Ultraschallwellen auf der Oberfläche (S; LF) des Objekts zurückzuführen sind,

-- Korrigieren der Sendeverzögerungen der Schallgeber ($114_1$, ..., $114_N$) mit Hilfe der Signale von Zwischenmesssignalen (M) und Steuern der Schallgeber ($114_1$, ..., $114_N$), damit sie zu der Oberfläche (S; LF) des Objekts Ultraschallwellen senden, die zueinander korrigierte Sendeverzögerungen ($L^p$) aufweisen,

- Empfangen von den Schallgebern ($114_1$, ..., $114_N$) der abschließenden Messsignale (M), die aus der Reflexion einer Wellenfront resultieren, die gleichzeitig auf der Oberfläche (S; LF) des Objekts empfangen wird,

**dadurch gekennzeichnet, dass** die Steuer- und Verarbeitungsmittel (116) außerdem Folgendes umfassen:

- Mittel zum Bestimmen der Flugzeiten, Spiegelungsflugzeiten genannt, und die jeweils der Mindestzeit entsprechen, die ein von einem Schallgeber gesendetes Signal braucht, um zu diesem Schallgeber in Form eines Echos im Anschluss an eine Reflexion an der Oberfläche des Objekts zurückzukehren, zwischen jedem Schallgeber ($114_1$, ..., $114_N$) und der Oberfläche (S; LF) des Objekts ausgehend von den abschließenden Messsignalen (M) und den korrigierten Sendeverzögerungen ($L^f$), und

- Mittel zur geometrischen Rekonstitution der Oberfläche (S; LF) des Objekts ausgehend von den bestimmten Spiegelungsflugzeiten.

## Claims

1. A method for reconstructing the geometry of a surface (S; LF) of an object (102; 102') via echographic probing using an ultrasound probe (106) provided with a plurality of transducers ($114_1$, ..., $114_N$), comprising the following steps:

- controlling ($204_1$, ..., $204_N$) the transducers ($114_1$, ..., $114_N$) such that they transmit towards said surface (S; LF) of the object ultrasound waves having initial transmission delays ($L^0$) in relation to one another,

- executing at least once the following cycle of steps, in such a way as to obtain after at least one iteration a wavefront received simultaneously on the surface:

• receiving ($212_1$, ..., $212_N$) from the transducers ($114_1$, ..., $114_N$) intermediate measurement signals (M), measuring in particular echoes due to reflections of the ultrasound waves on said surface (S; LF) of the object,

• correcting (202) the transmission delays of the transducers ($114_1$, ..., $114_N$) using the intermediate measurement signals (M) and controlling ($204_1$, ..., $204_N$) the transducers ($114_1$, ..., $114_N$) such that they transmit towards said surface (S; LF) of the object ultrasound waves having the corrected transmission delays ($L^p$) in relation to one another,

- receiving ($212_1$, ..., $212_N$) from the transducers ($114_1$, ..., $114_N$) final measurement signals (M) resulting from the reflection of a wavefront received simultaneously on said surface (S; LF) of the object,

**characterized in that** it further comprises the following steps:

- determining (226) specular travel times, each corresponding to the minimum time taken by a signal transmitted by a transducer to return to this transducer in the form of an echo following a reflection against the surface of the object, between each transducer ($114_1$, ..., $114_N$) and said surface (S; LF) of the object on the basis of the final measurement signals (M) and of the corrected transmission delays ($L^f$), and

- geometric reconstitution (228, 230, 232) of said surface (S; LF) of the object on the basis of the determined specular travel times.

2. The method for reconstructing a surface geometry (S; LF) as claimed in claim 1, wherein the determining (226) of the specular travel times comprises:

- determining a roundtrip travel time, common to all of the transducers ($114_1$, ..., $114_N$), of the wavefront received simultaneously on said surface (S; LF) of the object, and

- calculating specular travel times on the basis of this roundtrip travel time, the corrected transmission delays ($L^f$) and reception shifts ($R^f$) applied to the transducers.

3. The method for reconstructing a surface geometry (S; LF) as claimed in claim 1 or 2, wherein the geometric reconstitution (228, 230, 232) of said surface (S; LF) comprises:

   - determining (228) distances ($d_n$) separating each transducer ($114_1$, ..., $114_N$) from said surface (S; LF) on the basis of determined specular travel times,
   - calculating (230) coordinates of points ($P_n$) of said surface (S; LF) according to the coordinates ($c_n$) of the transducers ($114_1$, ..., $114_N$) and of the determined distances ($d_n$), and
   - the geometric reconstitution (232) of said surface (S; LF) via interpolation between these points ($P_n$).

4. The method for reconstructing a surface geometry (S; LF) as claimed in claim 3, wherein the calculating (230) of the coordinates of points ($P_n$) of said surface (S; LF) is based on the hypothesis that said surface is tangent to a set of spheres centered respectively on the transducers ($114_1$, ..., $114_N$) and with respective radiuses corresponding to the determined distances ($d_n$).

5. The method for reconstructing a surface geometry (S; LF) as claimed in claim 3 or 4, wherein the transducers ($114_1$, ..., $114_N$) are arranged linearly and the geometric reconstitution (228, 230, 232) of said surface (S; LF) comprises the reconstitution of a profile of this surface in the test plane of the probe.

6. The method for reconstructing a surface geometry (S; LF) as claimed in claim 5, wherein N transducers ($114_1$, ..., $114_N$) are arranged linearly, with the coordinates of their centers able to be expressed in the form ($c_n$, 0) in a reference frame (O, x, y) linked to the probe (106), and wherein the coordinates ($x_n$, $y_n$) of said points ($P_n$) of said surface (S; LF) are calculated using the following relationship:

$$\forall n,\ 1 \leq n \leq N\text{-}1,\ \begin{cases} x_n = c_n - d_n \cdot d_n{}' \\ y_n = d_n \sqrt{1 - d_n{}'} \end{cases},\ \text{where } d_n{}' = \frac{d_{n+1} - d_n}{c_{n+1} - c_n}\ \text{and } d_n \text{ designates the distance separating the}$$

n-th transducer from said surface.

7. The method for reconstructing a surface geometry (S; LF) as claimed in claim 3 or 4, wherein the transducers ($114_1$, ..., $114_N$) are arranged two-dimensionally in a matrix and the geometric reconstitution (228, 230, 232) of said surface (S; LF) comprises a three-dimensional reconstitution of this surface.

8. The method for reconstructing a surface geometry (S; LF) as claimed in any of claims 3 to 7, wherein the geometric reconstitution (228, 230, 232) of said surface (S; LF) is accomplished by linear or bilinear interpolation between said points ($P_n$).

9. A computer program (122) that can be downloaded from a communications network and/or recorded on a support (120) that can be read by a computer and/or can be executed by a processor (118), **characterized in that** it comprises instructions for the execution of steps of a method for reconstructing the geometry of a surface of an object (102; 102') via echographic probing as claimed in any of claims 1 to 8, when said program is executed on a computer.

10. An ultrasound probing device comprising a probe (106) comprising a case (112) and a plurality of ultrasound transducers ($114_1$, ..., $114_N$) attached to the case (112), and means for controlling and for processing (116) designed for:

   - controlling the transducers ($114_1$, ..., $114_N$) such that they transmit towards said surface (S; LF) of the object ultrasound waves having initial transmission delays ($L^0$) in relation to one another,
   - executing at least once the following cycle of steps, in such a way as to obtain after at least one iteration a wavefront received simultaneously on the surface:

      • receiving from the transducers ($114_1$, ..., $114_N$) intermediate measurement signals (M), measuring in particular echoes due to reflections of the ultrasound waves on said surface (S; LF) of the object,
      • correcting the transmission delays of the transducers ($114_1$, ..., $114_N$) using the intermediate measurement signals (M) and controlling the transducers ($114_1$, ..., $114_N$) such that they transmit towards said surface (S; LF) of the object ultrasound waves having the corrected transmission delays ($L^p$) in relation to one another,

   - receiving from the transducers ($114_1$, ..., $114_N$) final measurement signals (M) resulting from the reflection of a wavefront received simultaneously on said surface (S; LF) of the object,

**characterized in that** the means for controlling and for processing (116) further comprise:

- means for determining specular travel times, each corresponding to the minimum time taken by a signal transmitted by a transducer to return to this transducer in the form of an echo following a reflection against the surface of the object, between each transducer ($114_1$, ..., $114_N$) and said surface (S; LF) of the object on the basis of the final measurement signals (M) and of the corrected transmission delays ($L^f$), and
- means for the geometric reconstitution of said surface (S; LF) of the object on the basis of the determined specular travel times.

## Figure 1

_Figure 2_

*Figure 3*

*Figure 4*

*Figure 5*

*Figure 6*

*Figure 7*

## Figure 8A

$L^f$

## Figure 8B

$$\forall n,\ t_n = Tc - L_n^{\,f} + R_n^{\,f}$$

$R^f$

106

$d_n$

102

## Figure 8C

## Figure 9

106

O

x

y

$c_n$    $c_{n+1}$

$d_n$    $d_{n+1}$

$P_{n+1}$

$P_n$

102

*Figure 10*

LR

106

S

D

102

*Figure 11*

106

102

$114_1, ..., 114_N$

S

## *Figure 12*

## *Figure 13*

## Figure 14

## Figure 15

## Figure 16

## Figure 17

## RÉFÉRENCES CITÉES DANS LA DESCRIPTION

**Documents brevets cités dans la description**

- US 20060195273 A1

- FR 2786651

**Littérature non-brevet citée dans la description**

- **S. ROBERT et al.** Méthodes ultrasonores de détection et d'imagerie temps-réel implémentées dans les systèmes d'acquisition M2M. *conférences et exposition sur les essais non destructifs,* 24 Mai 2011
- **C. HOLMES et al.** Post-processing of the full matrix of ultrasonic transmit-receive array data for non-destructive évaluation. *NDT & E International,* Décembre 2005, vol. 38 (8), 701-711

- **A. FIDAHOUSSEN et al.** Imaging of defects in several complex configurations by simulation-helped processing of ultrasonic array data. *36th Annual Review of Progress in Quantitative Non-Destructive Evaluation,* 2010, vol. 29, 847-854
- **A. BAZULIN et al.** Algorithms and software development for welds automated ultrasonic inspection basing on phased arrays. *la 10e European Conférence on Non-Destructive Testing,* 07 Juin 2010